# EUROPÄISCHE PATENTSCHRIFT

(11) **EP 0 393 684 B1**
(45) Veröffentlichungstag und Bekanntmachung des Hinweises auf die Patenterteilung: **18.06.1997**
(21) Anmeldenummer: 90107465.8
(22) Anmeldetag: 19.04.1990
(51) Int. Cl.: C12N 15/70, C12N 15/74

(54) **Rekombinante DNA und Expressionsvektor**
Recombinant DNA and expression vector
ADN recombinant et vecteur d'expression

(30) Priorität: 21.04.1989 DE 3913201; 07.08.1989 DE 3926076
(43) Veröffentlichungstag der Anmeldung: 24.10.1990
(73) Patentinhaber: BOEHRINGER MANNHEIM GMBH, 68298 Mannheim (DE)
(72) Erfinder: Böck, August, Prof. Dr., D-8085 Geltendorf 2 (DE); Sawers, Robert Gary, Dr. rer. nat., D-8000 München 19 (DE); Jarsch, Michael, Dr. rer. nat., D-8173 Bad Heilbrunn (DE); Herbst, Roland, D-8000 München 40 (DE)
(74) Vertreter: Huber, Bernhard, Dipl.-Chem.

(56) Entgegenhaltungen:
- EP-A- 0 285 152
- JOURNAL OF BACTERIOLOGY, Band 170, Nr. 11, November 1988, Seiten 5330-5336, American Society for Microbiology; G. SAWERS et al.: "Anaerobic regulation of pyruvate formate-lyase from Escherichia coli K-12"
- JOURNAL OF BACTERIOLOGY, Band 171, Nr. 5, Mai 1989, Seiten 2485-2498, American Society of Microbiology; G. SAWERS et al.: "Novel transcriptional control of the pyruvate formate-lyase gene: upstream regulatory sequences and multiple promoters regulate anaerobic expression".

## Beschreibung

Die Erfindung betrifft rekombinante DNA und Expressionsvektoren, Verfahren zur Herstellung solcher rekombinanter DNA und Expressionsvektoren sowie deren Verwendung zur induzierbaren und reprimierbaren Expression eines Fremdgens.

Ein wichtiges Ziel der angewandten Gentechnologie ist die Proteinproduktion aus rekombinanter DNA. Dazu wird eine besondere Klasse von Vektoren, die sog. Expressionsvektoren, benötigt. Diese besitzen nicht nur die strukturellen Voraussetzungen für die Klonierung, den Transfer und die Vermehrung der rekombinanten DNA, sondern auch für die Expression des Proteins. Hierzu enthalten diese rekombinanten DNA-Moleküle spezielle Regulationssequenzen, die Promotoren, welche die Transkription der DNA-Sequenz in RNA bewirken, deren Translation dann durch die Ribosomen zum fertigen Protein führt.

Als Promotoren werden DNA-Regionen bezeichnet, an die bakterielle RNA-Polymerase zur Transkription eines oder mehrerer Gene bindet. Viele solcher Promotoren haben strukturelle Gemeinsamkeiten, deren Bedeutung u.a. in der Interaktion mit bestimmten Proteinen vermutet wird. Durch solche Interaktionen mit zellulären Proteinen oder anderen Molekülen, kann eine Repression, jedoch auch eine Induktion der Aktivität eines Promotors bewirkt werden. Ein Beispiel dafür ist die Interaktion des Lambda-Promotors P₁ mit dem Lambda-Repressor cI.

Bei der gentechnologischen Produktion von Proteinen ist es von besonderem Vorteil, wenn ein in einem Expressionsvektor vorhandener Promotor durch Vorhandensein oder Zugabe von Repressor oder Induktor reguliert werden kann.

Diese Regulation dann dadurch erfolgen, daß zu Beginn der Fermentation die Aktivität des Promotors unterdrückt wird, so daß unter minimaler Beeinträchtigung der Vitalität der Zellen eine hohe Biomasse produziert werden kann. Anschließend wird durch geeignete Maßnahmen der Promotor stimuliert und die Synthese des Produkts kann erfolgen. Prinzipiell läßt sich also der Fermentationsprozeß in eine Wachstums- und eine Produktionsphase unterscheiden.

Für die regulierbare Genexpression finden beispielsweise der PL-Promotor des Bakteriophagen Lambda, der lac-Promotor, der trp-Promotor, der tac-Promotor, der trc-Promotor und der rac-Promotor Anwendung.

Diese Expressionssysteme sind jedoch für eine großtechnische Anwendung nur bedingt geeignet. Vor allem eine Temperaturerhöhung auf 42°C, wie sie zur Induktion des Lambda PL-Promotors erforderlich ist, ist bei einer technischen Anwendung mit Volumen ab 50 l mit großen Schwierigkeiten verbunden. Außerdem hat sich gezeigt, daß die Induktion des Lambda PL-Promotors in einer frühen Wachstumsphase erfolgen muß, so daß die für eine Großproduktion erforderliche Biomasse unter Umständen nicht erreicht werden kann.

Bei Verwendung des lac-Promotors für Expressionsvektoren ist es im Gegensatz zum PL-Promotor nicht möglich, das System mit Hilfe einer Kopie des Repressor-Gens vollständig zu reprimieren, da durch die Kopienzahl des lac-Operators der Repressor austitriert wird. Zwar läßt sich die Repression durch Verwendung von Repressor-Überproduzenten wieder herstellen, aber solche Stämme sind nur partiell induzierbar. Wenn das entsprechende Repressorgen nicht mit auf dem Expressionsvektor enthalten ist, so ist man beim Lambda PL, beim lac und den anderen Derivaten des lac-Promotors in der Wahl der Wirtsstämme eingeschränkt. Außerdem ist die bei diesen Systemen notwendige Zugabe von Induktoren während der Fermentation nicht nur teuer, sondern bereitet auch prinzipielle Schwierigkeiten, vor allem, wenn es sich um metabolisierbare Induktoren, z.B. Lactose, handelt.

Auch beim trp-Promotor handelt es sich um ein System, das sich nicht vollständig reprimieren läßt. Wie bei der Verwendung von Induktoren, so wird auch durch Zugabe von Tryptophan zur Repression die Fermentation zusätzlich wesentlich verteuert.

Die bisher bekannten Induktoren sind demnach nur beschränkt für den industriellen Einsatz geeignet, da diese Induktoren meist sehr teuer und die angewandten Verfahren zur Induktion oder Repression kompliziert und nur bedingt zur Regulation der Expression eines Proteins geeignet sind.

Aus der DE-A 37 10 633 ist ein sauerstoffreprimierbarer Promotor bekannt, der aus dem FdhF-Gen stammt und mit Formiat induzierbar ist. Damit ist zwar die Möglichkeit für eine einfache Repression und Induktion geschaffen, jedoch handelt es sich hierbei um einen relativ schwachen Promotor.

Es ist daher Aufgabe dieser Erfindung, rekombinante DNA und Expressionsvektoren bereitzustellen, die auf einfache Art und Weise eine Regulation der Expression eines gewünschten Genproduktes sowie eine besonders hohe Expressionsrate der Gene ermöglichen.

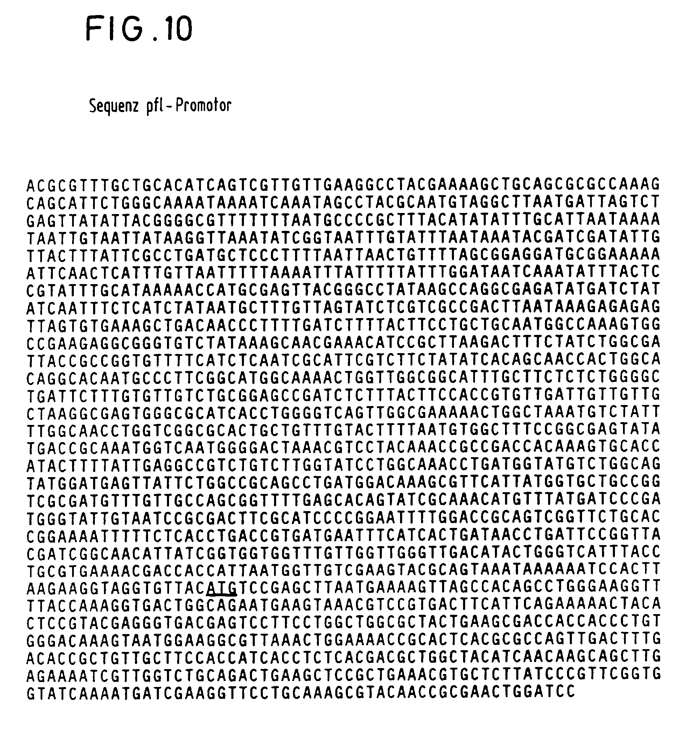

Gelöst wird diese Aufgabe durch eine rekombinante DNA, die dadurch gekennzeichnet ist, daß sie enthält:
a) eine Regulatorregion, die zu mindestens 50 % homolog ist zu den Teilbereichen -969 bis -991 Basenpaare und/oder -1308 bis -1330 Basenpaare der Sequenz von Figur 10 und
b) in der 3'-Richtung von der Regulationssequenz eine Promotorregion, welche eine -35/-10-Promotor-KonsensusSequenz (Rosenberg, M. and Court, D. (1979) Ann.Rev. Genet. 13: 319-353) aufweist.

In einer bevorzugten Ausführungsform der Erfindung ist die Regulatorregion zu mindestens 65 % homolog zu den genannten Sequenzen der Figur 10.

Die Numerierung der Basen ist hierbei auf das in Figur 10 unterstrichene ATG-Startkodon des pfl-Gens als +1 (für Adenin) bezogen. Auf der 5'-Seite hiervon liegende Nukleotide haben negative Numerierungen.

In einer weiteren bevorzugten Ausführungsform der Erfindung enthält die rekombinante DNA zusätzlich mindestens eine dritte Sequenz, die mindestens zu 80 % homolog zu der folgenden Konsensussequenz ist:

      C   T
TATTTG AT AA
      G   -

Diese dritte Sequenz kann hierbei einfach oder mehrfach in der rekombinanten DNA enthalten sein.

Geeignete Promotoren der Promotorregion der erfindungsgemäßen rekombinanten DNA sind alle Promotoren, die eine KonsensusSequenz in der -35/-10-Region, wie oben definiert, enthalten. Dies sind z.B. lac-Promotor, Lambda-PL-Promotor, trp-Promotor, mgl-Promotor (EPA 285152) oder die Promotoren aus Figur 11 bzw. deren zu 50 und vorzugsweise 65 % Homologe.

Vorzugsweise wird als Promotorregion eine Sequenz verwendet, welche zu 50 % und besonders bevorzugt zu 65 % homolog zu einer der in Figur II angegebenen Sequenzen ist.

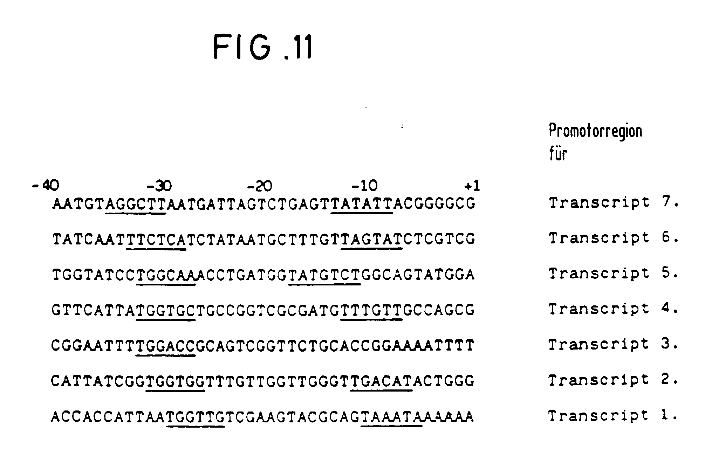

In einer ganz besonders bevorzugten Ausführungsform verwendet man mindestens einen der Promotoren von Transkript 6 und Transkript 7 der in Figur 11 gezeigten Sequenzen.

Ein weiterer Gegenstand der Erfindung ist ein Expressionsvektor, der eine erfindungsgemäße rekombinante DNA einligiert in einen geeigneten Vektor enthält.

Diese DNA-Sequenzen der erfindungsgemäßen rekombinanten DNA-Sequenz befinden sich im erfindungsgemäßen Expressionsvektor upstream (d.h. auf der 5'-Seite) vom Transkriptionsstart eines unter der Kontrolle dieses Promotors zu exprimierenden Gens, wobei sich zwischen Promotor und zu exprimierendem Gen ein ATG-Kodon und vorzugsweise noch eine Shine-DalgarnoSequenz befinden.

In einer bevorzugten Ausführungsform der Erfindung enthält der erfindungsgemäße Expressionsvektor an der Stelle, an der ein zu exprimierendes Fremdgen eingesetzt werden soll, einen Polylinker oder eine singuläre Restriktionsschnittstelle, d.h. eine Restriktionsschnittstelle, die in dem Expressionsvektor nur einmal vorhanden ist.

In einer weiteren bevorzugten Ausführungsform sind zwischen Promotorregion und zu exprimierenden Fremdgen das PFL-Gen bzw. Teile davon und gegebenenfalls untranslatierte upstream-Bereiche des PFL-Gens, die z.B. das ATG-Kodon und die Shine-Dalgarno-Sequenz enthalten, vorhanden. Besonders bevorzugt wird die in Figur 10 angegebene Sequenz, vorzugsweise die gesamte Sequenz, hierfür verwendet. In einer anderen bevorzugten Ausführungsform enthält der Expressionsvektor die erfindungsgemäße rekombinante DNA, untranslatierte Sequenzen aus dem upstream-Bereich des PFL-Gens von Figur 10 sowie Shine-Dalgarno-Sequenz und ATG des Fremdgens und gegebenenfalls bereits das Fremdgen selbst. Es ist jedoch erfindungsgemäß auch möglich, das Fremdgen einschließlich seines Startkodons direkt an die erfindungsgemäße rekombinante DNA zu koppeln.

Ein weiterer Gegenstand der Erfindung ist ein Verfahren zur Herstellung einer erfindungsgemäßen rekombinanten DNA, bei dem man aus der Genbank eines Mikroorganismus, der das PFL-Gen enthält, dieses mit seinen upstream-Regionen isoliert, gegebenenfalls die davon nicht benötigten Teile nach an sich bekannten Methoden entfernt, und die gewünschten Sequenzen mit einer Promotorregion bzw. dritten Sequenzen verbindet.

Hierzu werden Ligation, Restriktion und Deletion von DNA-Sequenzen nach üblichen Methoden durchgeführt.

In einer bevorzugten Ausführungsform der Erfindung wird die DNA-Sequenz aus einer Genbank eines Mikroorganismus aus der Familie der Enterobacteriaceae und vorzugsweise aus E.coli isoliert.

Ein wiederum weiterer Gegenstand der Erfindung ist ein Verfahren zur Herstellung eines erfindungsgemäßen Expressionsvektors. Hierzu insertiert man die erfindungsgemäße rekombinante DNA und gegebenenfalls auch einen Polylinker, eine Shine-Dalgarno-Sequenz, ein Startkodon und/oder gewünschte weitere Sequenzen in einen geeigneten Vektor. Hierfür geeignete Vektormoleküle sind dem Fachmann bekannt, z.B. pBR322 oder Derivate davon.

Die erfindungsgemäße Verwendung einer rekombinanten DNA oder eines Expressionsvektors wie sie im Vorhergehenden beschrieben sind, zur induzierbaren und reprimierbaren Expression eines Fremdgens ist dadurch gekennzeichnet, daß die Induktion unter anaeroben Bedingungen und durch Pyruvat und die Repression durch Sauerstoff bewirkt wird.

Die Expression kann hierbei in geeigneten Mikroorganismen der Gattung Enterobacteriaceae, wie vorzugsweise E.coli und Salmonella oder anderen gramnegativen Bakterien, wie vorzugsweise Pseudomonas, oder in grampositiven Bakterien erfolgen.

Bevorzugt wird die Expression in einem Wirtsstamm durchgeführt, der FNR-positiv ist, der also ein funktionelles FNR-Genprodukt bildet. Bevorzugt ist dies E.coli FM 420, der bei der Deutschen Sammlung für Mikroorganismen, DSM 5312, hinterlegt wurde.

Das FNR-Protein (Stewart, V. Microbiol.Rev. 52 (1988) 190-232), welches von FNR-positiven Mikroorganismen produziert wird, ist ein dimeres Protein, das mit dem Operator des erfindungsgemäßen Promotors interagieren kann und dabei die Expression aktiviert.

Es ist zwar möglich, auch einen Wirtsstamm zu verwenden, der FNR-negativ ist, in diesem Fall muß jedoch das FNR-Protein der Zelle zugeführt werden, um eine Aktivierung zu erreichen.

Dazu kann das FNR-Gen in den erfindungsgemäßen Expressionsvektor, der auch das gewünschte Fremdgen trägt oder tragen wird, einligiert sein, wobei gleichzeitig mit der Expression des Fremdgens auch die Expression des FNR-Proteins erreicht wird. Ebenso kann auch das FNR-Gen auf einem zusätzlichen Vektor in den Wirtszellen vorliegen. In diesem Fall ist die Produktion des FNR-Proteins unabhängig von der Produktion des Fremdgens, wobei diese jedoch von dem FNR-Protein induziert wird. Diese Ausführungsform, also das Einbringen mindestens eines FNR-Gens auf einem separaten Vektor, ist daher erfindungsgemäß bevorzugt.

Durch die erfindungsgemäße rekombinante DNA und die erfindungsgemäßen Expressionsvektoren ist es auf einfache Art und Weise möglich, die Expression eines Fremdgens zu regulieren. So wird beispielsweise in der aeroben frühen Wachstumsphase des verwendeten Mikroorganismus eine störende Expression des Fremdgens unterdrückt. Beim Übergang in die späte logarithmische anaeroben Wachstumsphase wird die Expression dann durch vom Mikroorganismus gebildetes Pyruvat induziert und durch Zugabe von Pyruvat ins Wachstumsmedium eine Verstärkung der Expression erreicht. Eine weitere Vereinfachung stellt dar, daß in der anaeroben späten logarithmischen Wachstumsphase die Zellen bereits hochgewachsen sind und eine optimale Dichte erreicht haben. In diesem Fall tritt automatisch eine Sauerstofflimitierung ein, die fermentationstechnisch noch verstärkt oder reguliert werden kann. Durch die hohe Zelldichte der Mikroorganismen kann eine optimale Expression des Fremdgens erreicht werden.

Die Verwendung der erfindungsgemäßen rekombinanten DNA und Expressionsvektoren zur Produktion von Proteinen stellt daher eine kostengünstige und einfache Möglichkeit zur Regulation dar, da teure und komplizierte Induktionen (Induktorzugabe, Temperaturshift usw.) nicht mehr notwendig sind. Die folgenden Beispiele in Verbindung mit den Abbildungen der Zeichnung erläutern die Erfindung weiter.

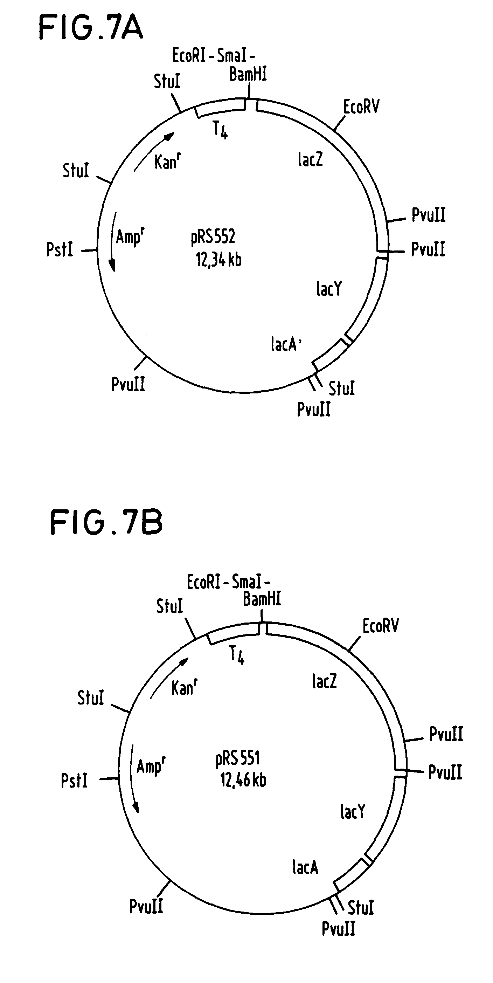

In der Zeichnung stellen dar:
- Fig. 1:: A) Auschnitt aus dem Plasmid p29:
   Kompletter regulatorischer Bereich und pfl-Strukturgen mit den anschließenden Terminatoren.
   fnr: Bindestelle für das fnr-GenProdukt; Tr: Transkriptionsstart; T: Terminator.
B) Ausschnitt aus dem Plasmid p29:
   Eingezeichnet sind die wichtigsten Schnittstellen für Restriktionsenzyme.
- Fig. 2:: Konstruktion des M13-Derivats M13pec23 (Erläuterung im Text)
- Fig. 3:: Schematische Darstellung der Deletionsmutagenese zur Kopplung des Creatinase-Gens an den pfl-Promotor; dargestellt am Beispiel M13pec23S.
Creatinase-Sequenzen sind schraffiert dargestellt; die EcoK-Kassette ist als schwarzer Balken dargestellt. (Erläuterung im Text)

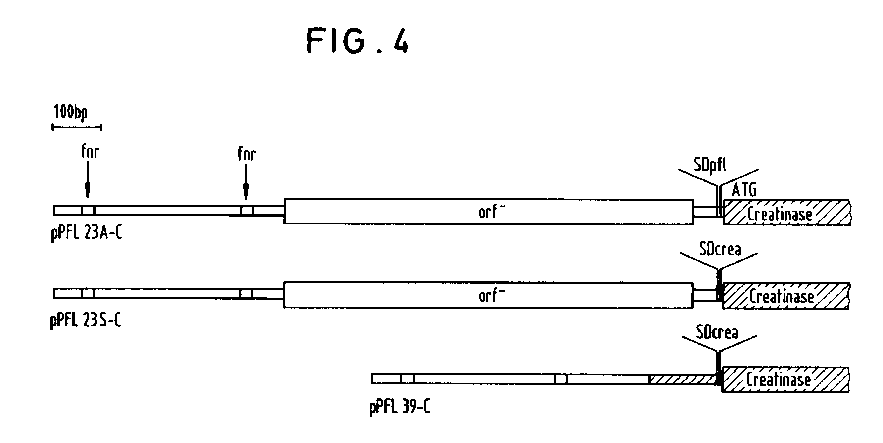

- Fig. 4:: Übersicht über die pfl-Creatinase-Fusionen: Dargestellt ist jeweils ein Ausschnitt aus den Plasmiden pPFL23S-C, pPFL23A-C und pPFL39-C.
SD: Shine-Dalgarno-Region.
- Fig. 5:: Umklonierung der pfl-Creatinase-Fusionen aus M13 in das Plasmid pGH-C, am Beispiel von M13pc23S.

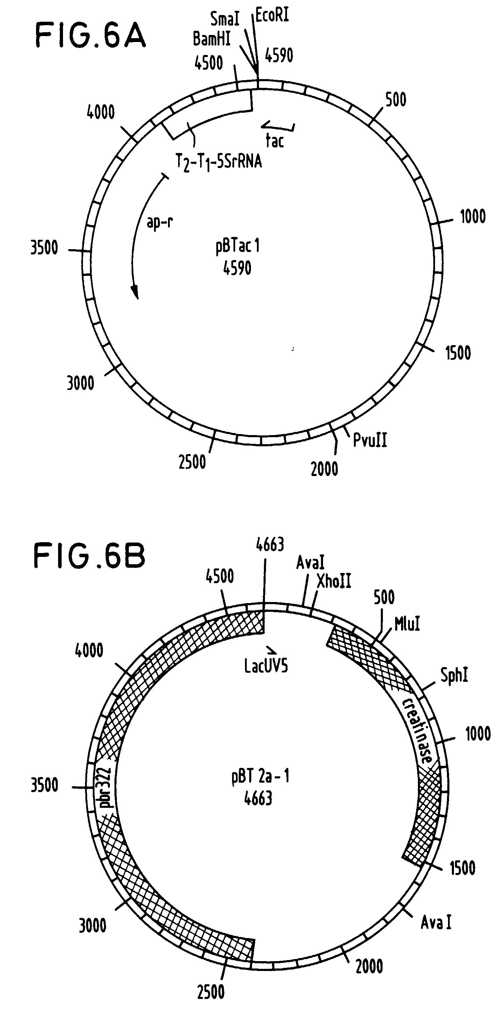

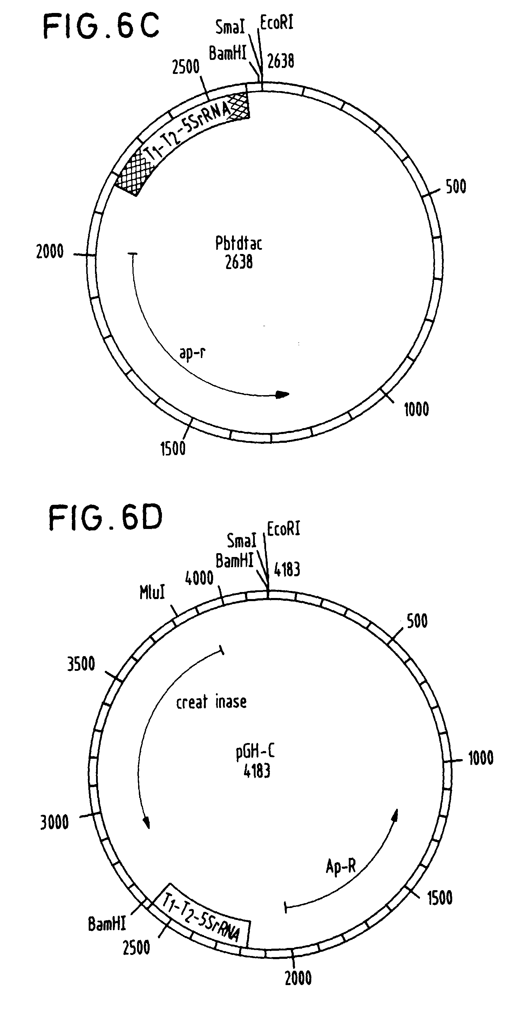

- Fig. 6:: A) Plasmidkarte von pBTacl
B) Plasmidkarte von pBT2a-1
C) Plasmidkarte von pBTdtac
D) Plasmidkarte von pGH-C
- Fig. 7:: A) Plasmidkarte von pRS552
B) Plasmidkarte von pRS551
- Fig. 8:: Konstruktion des Plasmids pRM23:
Translationale Kopplung des kompletten pfl-Promotorfragments an lacZ.
B: BamHI; E: EcoRI; S: SalI; H: HindIII; M: MluI;
P: PstI; Pv: PvuI.
- Fig. 9:: Kinetik eines Fermentationslaufs: FM420 (pPFL23S-C). Fermenter: Bioflo II (New Brunswick);
Füllvolumen: 4,01; Rührgeschwindigkeit konstant bei 300upm; Belüftung konstant mit 4,01/min;
Die Kurven geben wieder:
   - Verlauf des Wachstums (Zelldichte als log OD600);
   - Expression des Creatinase-Gens bei zunehmendem Mangel an Sauerstoff (Volumenaktivität:log Units/ml Medium);
   - Abnahme des Gehalts an Gelöstsauerstoff (%) im Verlauf des Wachstums.
- Fig.10:: Sequenz des pfl-Promotorbereichs. Das Startkodon des pfl-Strukturgens ist unterstrichen; die Position des ersten Nukleotids (A des ATG) wird mit +1 bezeichnet.
- Fig.11:: Promotorregionen 1 - 7

### Allgemeines zur Konstruktion der Expressionsplasmide

1.) Alle anaeroben Anzuchten wurden nach Balch und Wolfe (1976) in Serumflaschen durchgeführt. Aerobe Anzuchten erfolgten in Erlenmeyerkolben unter starkem Schütteln (die Kolben wurden max. mit 1/10 des angegebenen Volumens gefüllt). Die Kulturen wurden bei 37°C inkubiert.
2.) Medium: TGYEP (pH 6,5; 0,4% Glucose) (Begg et al., 1977).
3.) Transformation der verwendeten Stämme mit Plasmid-DNA erfolgte nach Standartprozeduren (Maniatis, 1982).
4.) Bestimmung der pfl-Enzymaktivität erfolgte nach Conradt et al. (1984).
5.) Bestimmung der Creatinase-Enzymaktivität erfolgte nach Schmitt (1984). Angegeben ist die spezifische Aktivität (U/mg Protein).
6.) Bestimmung der ß-Galaktosidase-Enzymaktivität erfolgte nach Miller (1972). Die Aktivitäten sind in Miller-units angegeben.
7.) Die Integration der pfl-lacZ-Fusionen in das Chromosom erfolgte gemäß der Methode von Simons et al. (1987). Ausgehend vom Stamm RM102 (fnr-, DSM 5311) wurden folgende Transduktanden erhalten: RM135, RM136, RM409, RM415, RM412. Ausgehend vom Stamm FM420 (fnr+, DSM 5312) wurden folgende Transduktanden erhalten: RM123, RM124, RM401, RM404, RM407.

Ausgangsvektoren:
1.) M13mp18 (Yanisch-Perron et al., 1985)
2.) M13k11Rx (Waye et al., 1985)
3.) pBT2a-1 (DSM 3148 P)
4.) p29 (Christiansen & Pedersen, 1981, DSM 5380)

Die Fusion des Promotors an das Creatinase-Strukturgen erfolgte durch gezielte Deletionsmutagense an der Einzelstrang-DNA eines M13-Konstruktes. Dieses enthält ein pfl-Fragment (regulatorische Sequenz und Beginn des Strukturgens), einen Selektionsmarker für die Mutagenese (EcoK-Kassette: enthält sequentiell 4 mal die Erkennungssequenz für das Restriktionssystem K aus E. coli) und ein Fragment des Creatinase-Gens (Beginn des Strukturgens mit einem Teil der 5'-untranslatierten Sequenz).

Für die pfl-lacZ-Fusionen (Beispiel 8 - 12) wurden die Plasmide p29 (Christiansen & Pedersen, 1981; DSM 5380), pRS551 (DSM 5382), pRS552 (DSM 5381) (Simons et al.,1987) verwendet.

### Beispiel 1

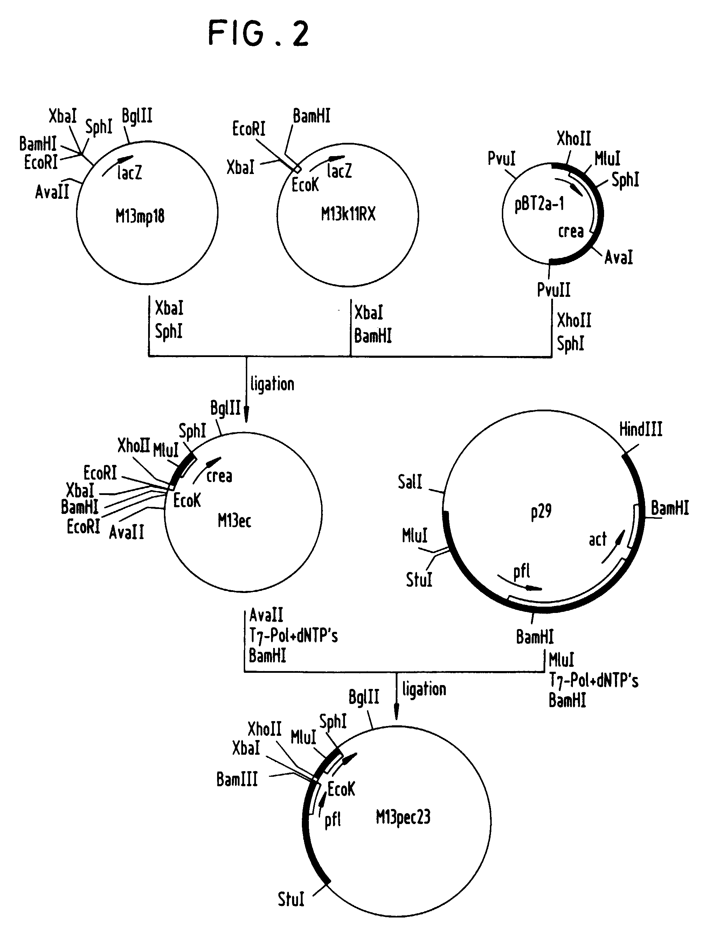

Klonierung der EcoK-Kassette und des Creatinase-Fragments in M13mp18
Zur Vorbereitung der Deletionsmutagenese wurden die einzelnen
Komponenten in M13mp18 kloniert. Im ersten Schritt wurden die
EcoK-Kassette und das Creatinase-Fragment in den XbaI/SphIgeschnittenen Vektor inseriert. Die EcoK-Kassette wurde als 90 bp XbaI/BaMHI-Fragment aus M13k11RX isoliert (Waye et al., 1985). Das Creatinase-Fragment wurde als 580bp XhoII/SphI-Fragment aus pBT2a-1 isoliert. Es enthält die ersten 460 Nukleotide des Strukturgens und 120 Nukleotide der 5'-untranslatierten Sequenz. Das 5'-überhängende Ende der XhoIISchnittstelle ist kompatibel mit dem überhängendem Ende der BaMHI-Schnittstelle (EcoK-Kassette). EcoK-Kassette, Creatinasefragment und Vektor wurden zusammengegeben und über die entsprechen-den Schnittstellen ligiert (Fig. 2). Transfiziert wurde E. coli RR1dM15 (rk-, mk-; ATCC 35102).
Das erhaltene Konstrukt wurde mit M13ec bezeichnet.

### Beispiel 2

### Klonierung des pfl-Promotor-Fragments in M13ec

Der Promotorbereich des pfl-Gens wurde als 1786bp MluI/BamHI-Fragment aus dem Plasmid p29 isoliert (pfl-Sequenz: +390 bis -1396, relativ zum ersten Nukleotid des pfl-Strukturgens, A des ATG von Fig. 10). p29 wurde mit MluI geschnitten, das 5'-überhängende Ende mit T7-Polymerase in Anwesenheit aller 4 dNTP's aufgefüllt und dann mit BamHI nachgeschnitten. Der Vektor M13ec wurde zuerst mit AvaII geschnitten, das überhängende Ende ebenfalls aufgefüllt und dann mit BamHI nachgeschnitten. Das isolierte pfl-Fragment wurde gerichtet in M13ec inseriert und das erhaltene Konstrukt mit M13pec23 bezeichnet (Abb. 2). Transfiziert wurde E. coli RR1dM15 (rk-, mk-; ATCC 35102). Die Orientierung des Creatinase-Fragments in M13pec23 entspricht der Transkriptionsrichtung vom pfl-Promotor.

### Beispiel 3

### Deletionsmutagense

Mit Hilfe von Oligonukleotiden wurden 2 Deletionsmutagenesen durchgeführt (analog der Methode von Waye et al., 1085), um das Creatinase- Strukturgen an den Promotor zu fusionieren. Es wurde je eine translationale Fusion (ersetzen des pfl-Strukturgens durch das Creatinase-Strukturgen ab dem Startkodon) und eine transkriptionale Fusion (Fusion des Creatinase-Gens mit eigener Shine-Dalgarno-Sequenz (SD-Sequenz) an den Promotor) hergestellt.
Diese wurden mit M13pc23A und M13pc23S bezeichnet (siehe Tabelle 1).

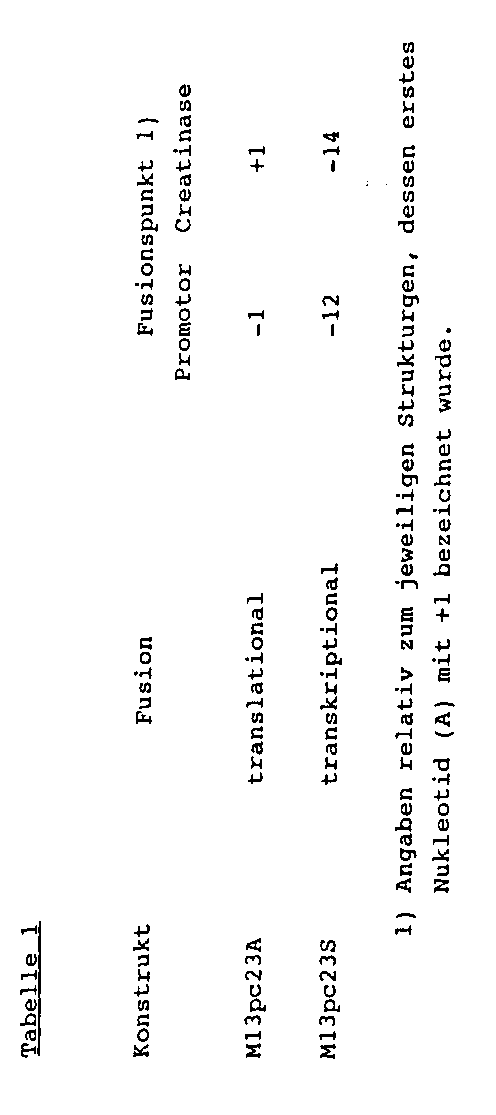

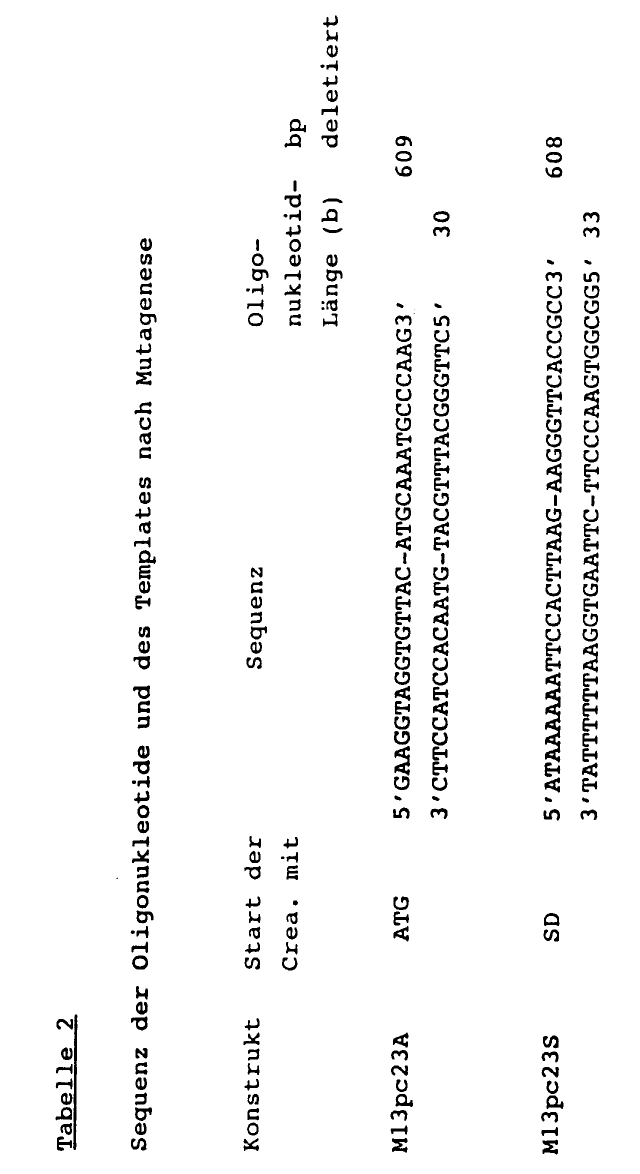

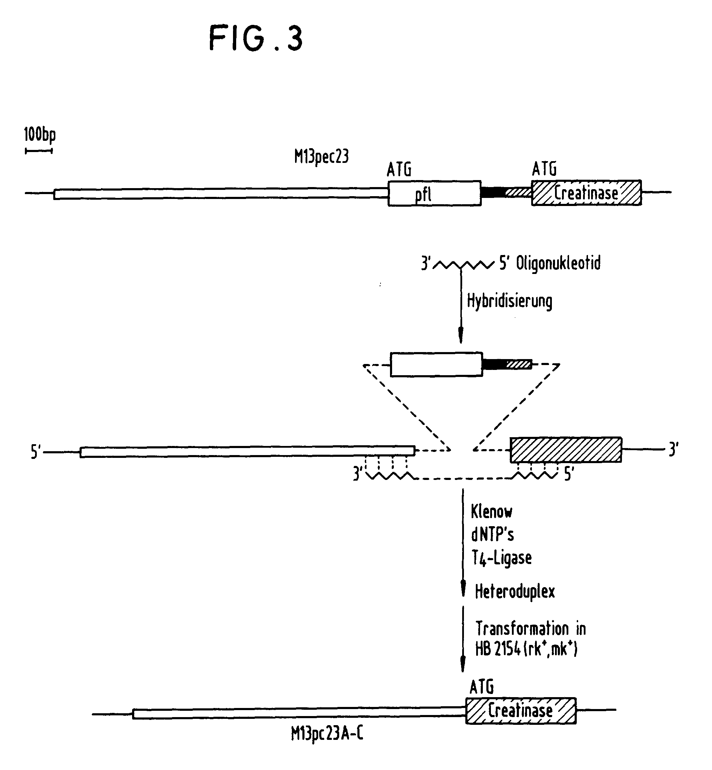

Durchführung der Mutagenese: (siehe Fig. 3)
a) Hybridisierung des mutagenen Oligonukleotids an den M13-Plus-Strang. Die zwischen den Fusionspunkten zu deletierende DNA-Sequenz bleibt ungepaart.
b) Der Rest der M13-Enzelstrang-DNA wird mit Klenow und dNTP's zum Doppelstrang aufgefüllt.
c) Die Selektion gegen den nicht mutierten Elternstrang erfolgt in vivo nach Transformation in E. coli JM83 (rk+, mk+; ATCC 35607).

### Beispiel 4

### Gezielte Mutagenese im orf-Gen

Um in den späteren Expressionsvektoren (highcopy Plasmide) eine Überexpression des orf-Genproduktes durch den Gendosiseffekt zu vermeiden, wurden durch gezielte Mutagenese nach Kunkel (Kunkel, 1985; Kunkel et al., 1987) zwei sequentielle Stopkodons in den Leserahmen des orf-Gens eingeführt.

Die Basensubstitutionen wurden mit einem mutagenen Oligonukleotid an der Plus-Strang-DNA des Konstruktes M13pc23S durchgeführt. Die ausgetauschten Basen und deren Positionen sind aus der folgenden Abbildung zu entnehmen:

5'..-...-AAC-TAG-TAA-...-...-...-...-3'
: ::
5'CG-AAA-AAC-TGG-CTA-AAT-GTC-TAT-TTT-3'
3'-TTT-TTG-ATC-ATT-TTA-CAG-A5'
*

Der mittlere Strang entspricht der Sequenz im Plus-Strang von M13pc23S (5'-->3'), der untere (3'-->5') der des mutagenen Oligonukleotids. Der oberste Strang ent-spricht der Sequenz nach Mutagenese; die ausgetauschten Basen sind durch Doppelpunkte (:) gekennzeichnet. Der Stern markiert die Position - 570 relativ zum pfl-Strukturgen.

An dem Konstrukt M13pc23A wurde keine Mutagenese durchgeführt. Hier wurde der pfl-Promotorbereich, inklusive des intakten orf-Gens, über die Schnittstellen AvaI und HindII entfernt und durch das entsprechende Segment aus der orfMutante von M13pc23S ersetzt.

### Beispiel 5

Konstruktion des promotorlosen Creatinase-Plasmids pGH-C
Ausgangsvektoren:
1.) pBTacl (Boehringer Mannheim GmbH, Best.-Nr.: 1081 365)
2.) pBT2a-1

pGH-C dient als Grundvektor zur Herstellung der Expressionsvektoren. Dieses Plasmid enthält das komplette Creatinase-Strukturgen und Terminations-sequenzen; durch Umklonierung der Fusionen aus den M13-Derivaten in pGH-C läßt sich die Expressionskassette vervollständigen.
a) Eliminierung des tac-Promotors aus pBTac1 Zur Eliminierung des tac-Promotors aus pBTac1 wurde das Plasmid mit EcoRI geschnitten und das 5'-überhängende Ende mit T7-Polymerase aufgefüllt. Anschließend wurde mit PvuII geschnitten und der Vektoranteil isoliert. Durch Ligation der glatten Enden wird die EcoRI-Schnittstelle regeneriert. Das erhaltene Konstrukt wurde mit pBTdtac bezeichnet.
b) Insertion des Creatinase-Gens in pBTdtac Das Creatinase-Gen wurde aus pBT2a-1 isoliert. Das Plasmid wurde mit AvaI geschnitten und das Fragment mit dem Creatinase-Gen (ca. 1600bp) isoliert . Die überhängenden Enden wurden aufgefüllt und mit BamHI-Linkern (BM) versehen. Das Fragment wurde in die BamHI-Schnittstelle von pBTdtac inseriert.
   Das erhaltene Konstrukt wurde mit pGH-C bezeichnet.

### Beispiel 6

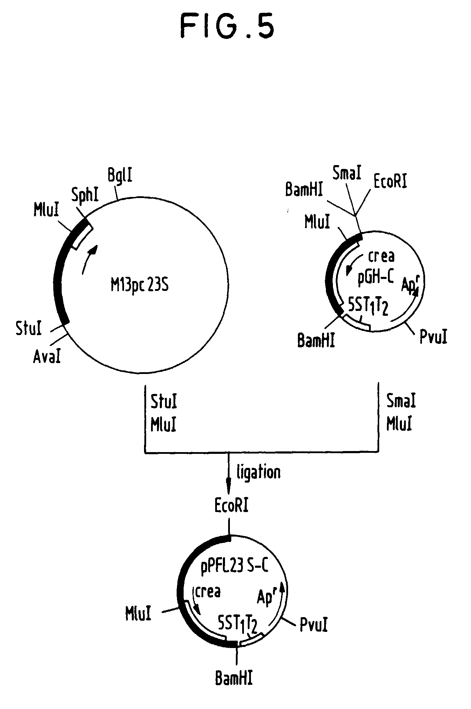

Umklonierung der Fusionen aus M13 in pGH-C
Die Fusionsfragmente aus M13pc23S und M13pc23A wurden über die Schnittstellen StuI und MluI isoliert.
Das Plasmid pGH-C wurde mit SmaI und MluI geschnitten und der Vektoranteil isoliert. Die Fusionsfragmente wurden gerichtet in den Vektor inseriert, so daß die Transkriptionsrichtung vom pfl-Promotor der Orientierung des Creatinase-Gens entspricht (Fig. 5).
Die Expressionplasmide wurden entsprechend den M13-Konstrukten mit pPFL23S-C und pPFL23A-C bezeichnet.
Diese Vektoren enthalten folgende Anteile des pfl-Promotorbereichs:
pPFL23A Pos. -1 bis einschließlich -1364
pPFL23S Pos. -12 bis einschließlich -1364

### Beispiel 7

### Konstruktion des Expressionsvektors pPFL39-C

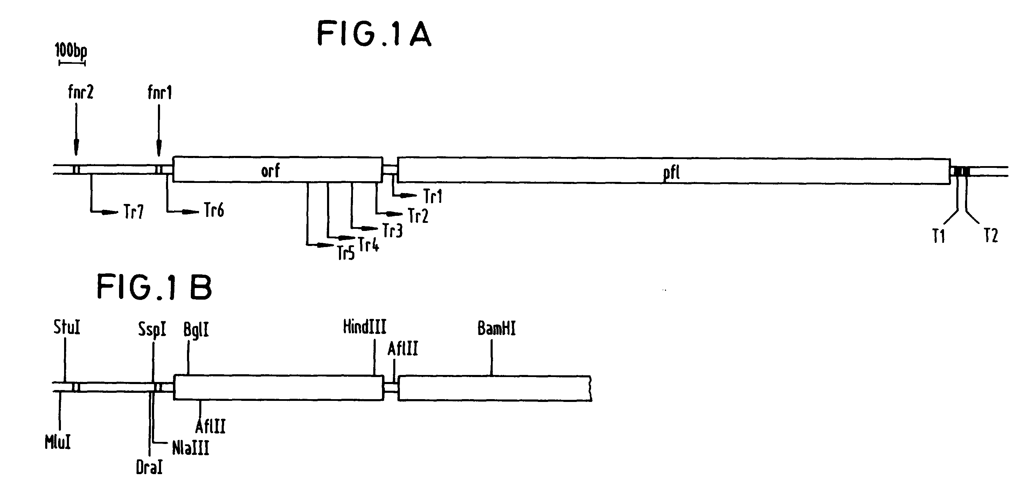

In diesem Plasmid ist nur ein verkürztes, 5'-seitiges Promotor-element enthalten (Promotoren 6 und 7, fnr-Boxen 1 und 2; Fig. 1).Dieses Element entspricht dem 577 bp MluI/AFlII-Fragment aus p29 und enthält die pfl-Sequenz von Position - 819 mit -1396 relativ zum pfl-Startkodon (A des ATG = +1). Das Fragment wurde über die EcoRI-Schnittstellen aus dem Plasmid pRM39 (Beispiel 10) isoliert und in die EcoRI-site des Plasmids pGH-C inseriert. Das erhaltene Konstrukt wurde mit pPFL-39C bezeichnet.

### Beispiel 8

### Konstruktion von pRM23

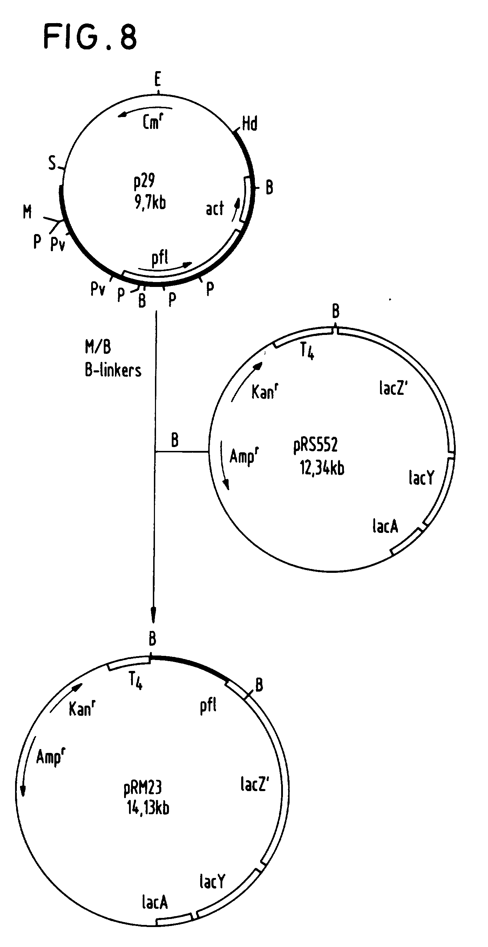

Translationale Kopplung des kompletten Promotorfragments mit dem lacZ-Gen.
Zur Isolierung des Promotorfragments wurde p29 mit MluI geschnitten und das überhängende Ende mit Klenow in Anwesenheit aller 4 dNTP's aufgefüllt. Anschließend wurde ein BamHI-Linker (8-mer) anligiert und mit BamHI geschnitten.
Das 1791bp-Fragment (inkl. BamHI-Linker) wurde isoliert und in die BamHI-Schnittstelle von pRS552 inseriert (Fig. 8).
Das pfl-Fragment besteht aus den Basen -1396 bis einschließlich +390 (relativ zum ersten Nukleotid des pfl-Strukturgens) und enthält die Promotoren 1 bis einschließlich 7 und die fnr-Boxen 1 und 2.

### Beispiel 9

### Konstruktion von pRM24

Translationale Kopplung eines verkürzten Promotorfragments mit dem lacZ-Gen.
Das Promotorelement wurde über die Schnittstellen SspI und BamHI aus p29 isoliert und über die Schnittstellen SmaI und BamHI in pRS552 inseriert. Das pfl-Fragment besteht aus den Basen -1045 bis einschließlich +390 (relativ zum pfl-Startkodon) und enthält die Promotoren 1 bis einschließlich 7 und die fnr-Box 2.

### Beispiel 10

### Konstruktion von pRM39

Transkriptionale Kopplung eines verkürzten, 5'-seitigen Promotorfragments mit dem lacZ-Gen.
Das Promotorelement wurde über die Schnittstellen MluI und AflII aus p29 isoliert, mit EcoRI-Linkern (10-mer) versehen und in die EcoRI-Schnittstelle von pRS551 inseriert.
Das pfl-Fragment besteht aus den Basen -819 bis einschließlich -1396 (relativ zum ersten Nukleotid des pfl-Strukturgens) und enthält die Promotoren 6 und 7 und die fnr-Boxen 1 und 2.

### Beispiel 11

### Konstruktion von pRM43

Transkriptionale Kopplung eines verkürzten, 5'-seitigen Promotorfragments mit dem lacZ-Gen.
Das Promotorelement wurde über die Schnittstellen MluI und DraI aus p29 isoliert, mit EcoRI-Linkern (10-mer) versehen und in die EcoRI-Schnittstelle von pRS551 inseriert.

Das pfl-Fragment besteht aus den Basen -1075 bis einschließlich -1396 (relativ zum ersten Nukleotid des pfl-Strukturgens) und enthält Promotor 7 und die fnr-Box 2.

### Beispiel 12

### Konstruktion von pRM46

Transkriptionale Kopplung eines verkürzten pfl-Fragments mit dem lacZ-Gen.
Das Promotorelement wurde über die Schnittstellen NlaIII und BglI aus p29 isoliert, mit EcoRI-Linkern versehen und in die EcoRI-Schnittstelle von pRS551 inseriert.
Das pfl-Fragment besteht aus den Basen -861 bis einschließlich -1016 (relativ zum ersten Nukleotid des pfl Strukturgens) und enthält Promotor 6 und die fnr-Box 1.

### Beispiel 13

### Expression von Pyruvat-Formiat-Lyase durch das komplette Promotorfragment (Promotor 1 - 7, fnr-Boxen 1 und 2): homologe Expression.

Angegeben ist der Anteil von Pyruvat-Formiat-Lyase am Gesamtzellprotein unter anaeroben Bedingungen.
Die Bestimmung erfolgte über die Ermittlung der spezifischen Aktivität der gebildeten Pyruvat-Formiat-Lyase.

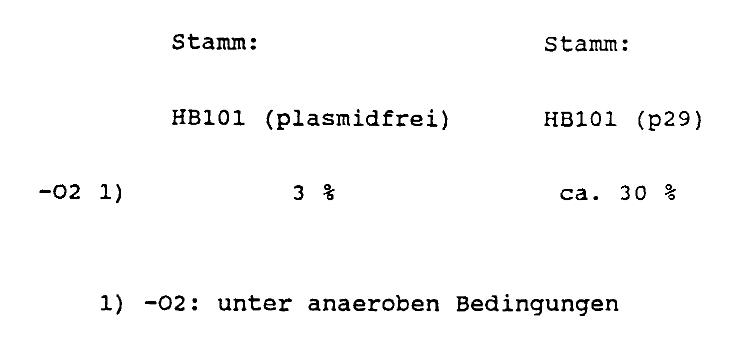

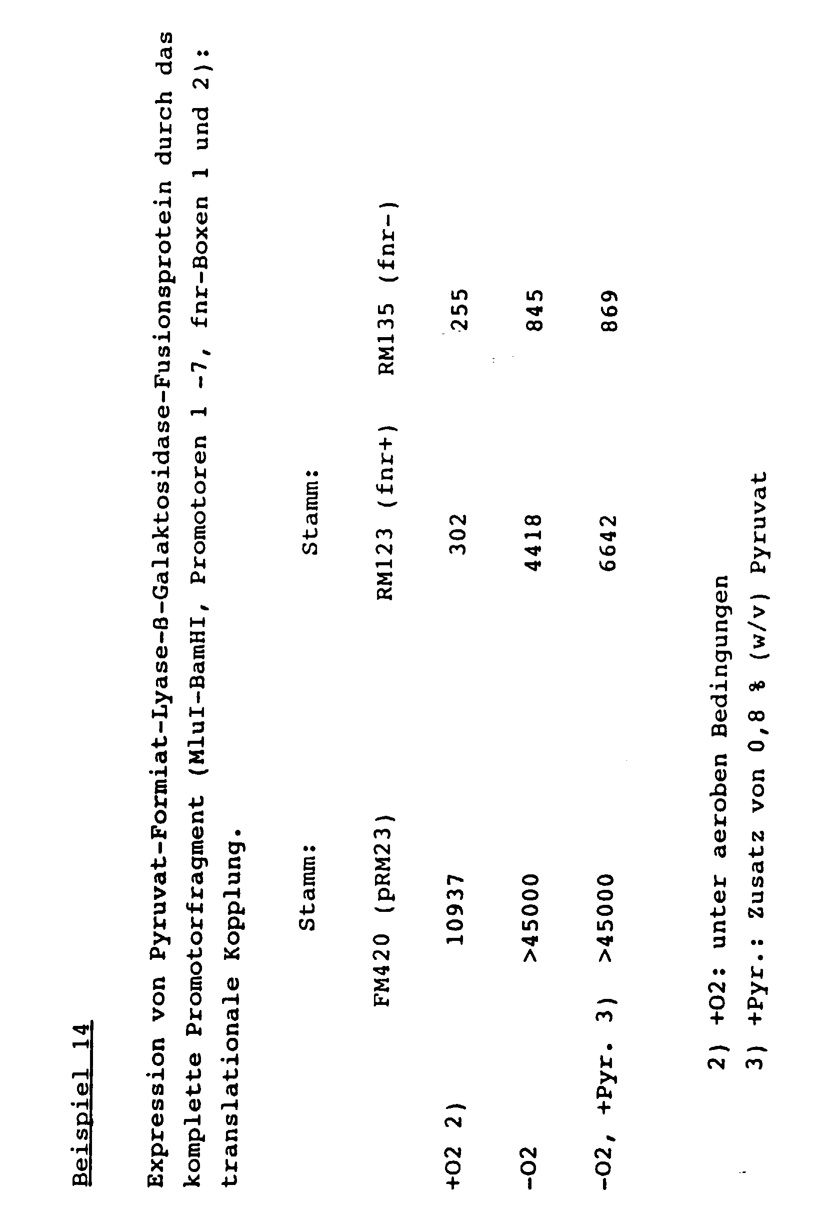

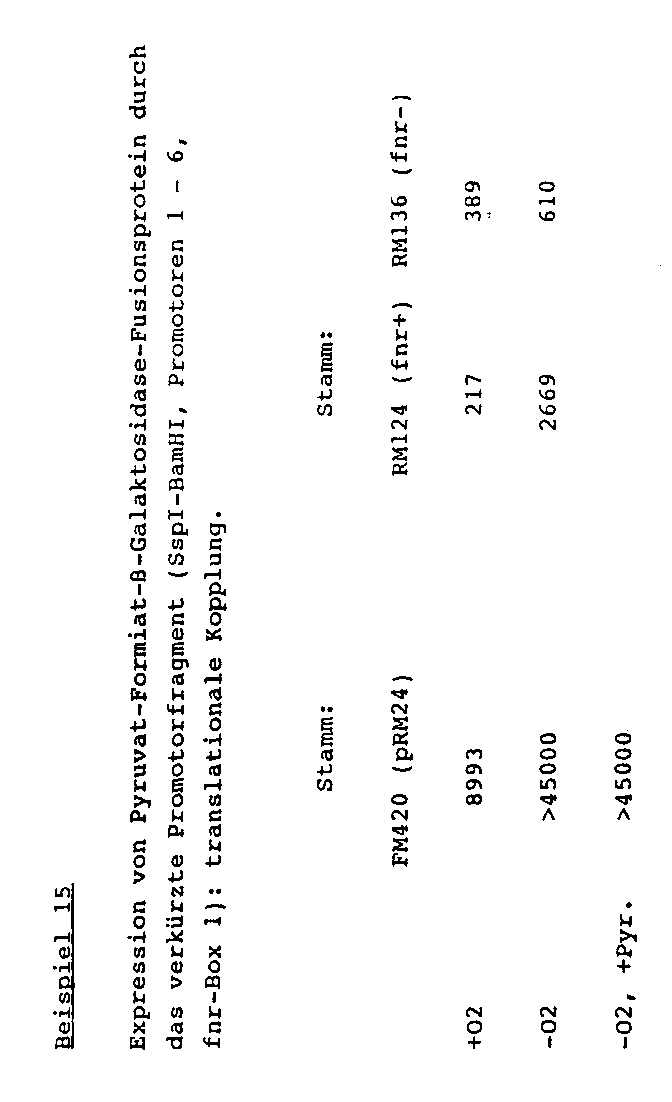

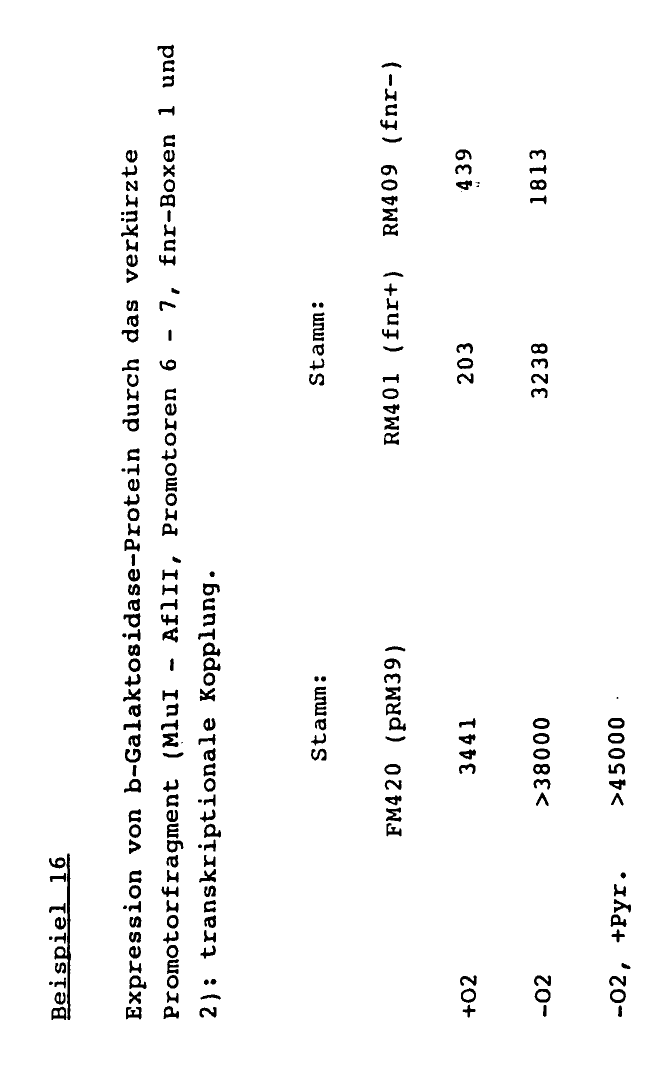

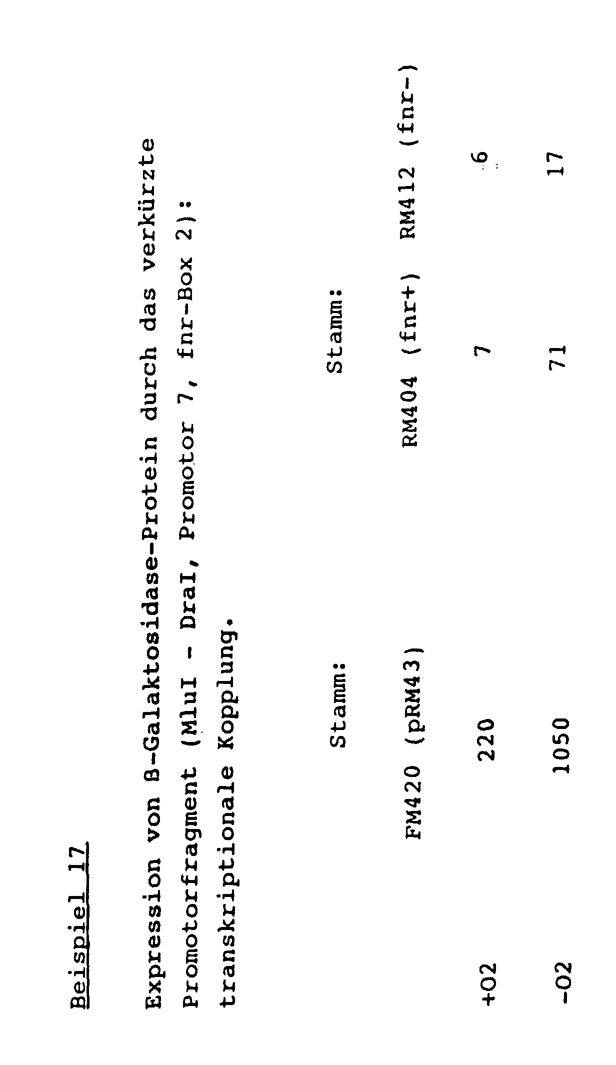

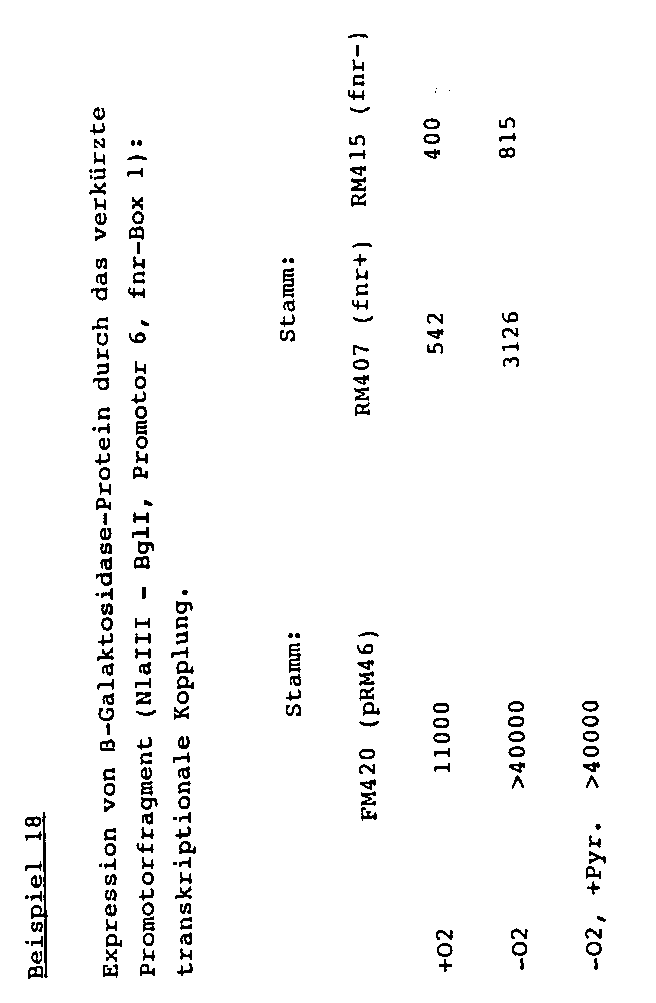

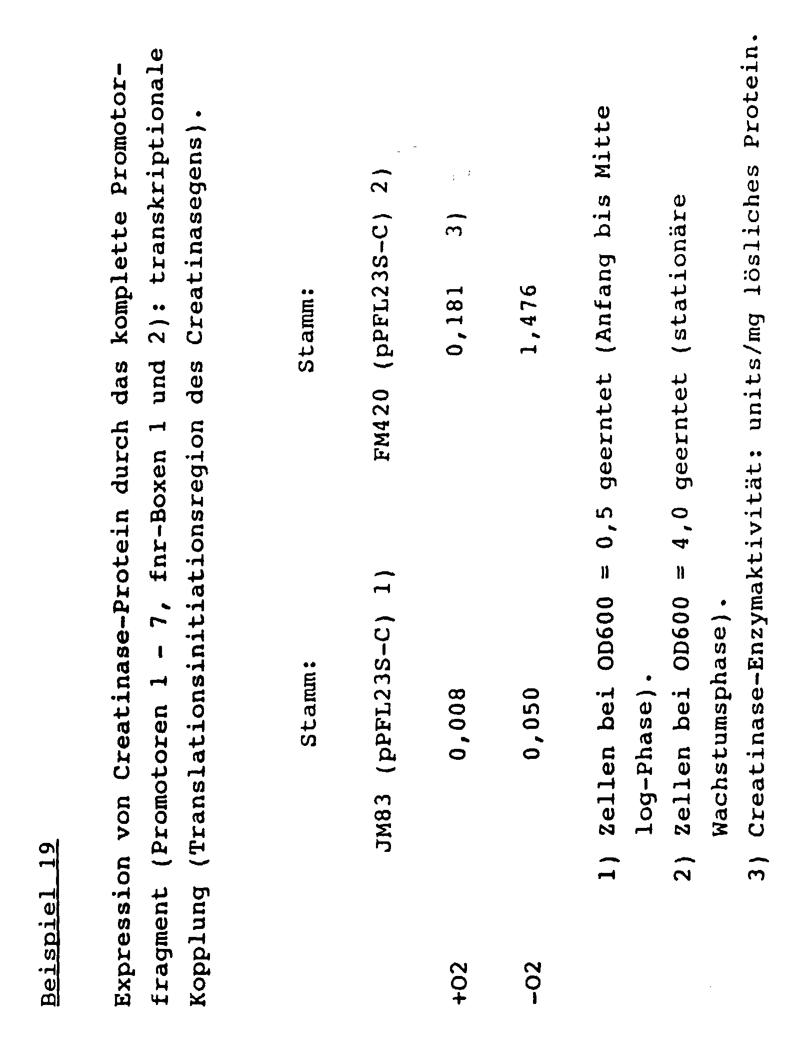

Fermentation von FM420 (pPFL23S-C):

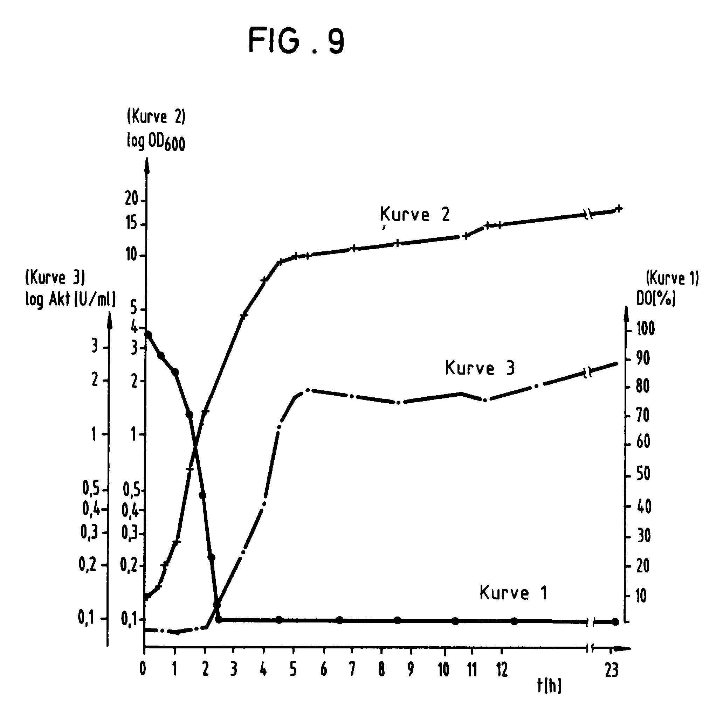

Fig. 9 gibt den Verlauf der Creatinase-Expression (Volumen-Aktivität: units/ml) im Verlauf eines Fermentationsprozesses wieder.
- Fermenter :: Bioflo II (New Brunswick, 5-Liter-Fermenter)
- Füllvol. :: 4 Liter
- Medium :: K2HPO4 (3 H20):8 g/Liter; KH2PO4:
2g/Liter
Pepton: 10 g/Liter; Hefeextrakt (Ohly Kav): 32,4g/Liter;
MgSO4 (1M): 4 ml/Liter; Glucose: 0,4 %.
- Temperatur :: 32°C
- pH :: 7,0 (konstant)
Rührgeschwindigkeit (300upm) und Belüftungsrate (41/min) wurden während des ganzen Laufs konstant gehalten.
Eingezeichnet sind:
- Wachstumskurve (Zelldichte als OD600).
- Creatinase-Expression (units/ml).
- Gehalt des Mediums an gelöstem Sauerstoff (DO = dissolved oxygen; %).

Der Grafik ist zu entnehmen:
- Abnahme des DO-Wertes mit Zunahme der Zelldichte.
- Kein negativer Einfluß auf das Wachstumsverhalten durch Eintreten sauerstofflimitierender Bedingungen (DO-Wert = 0).
- Induktion der Creatinase-Expression bei niedrigem DO-Wert.

### Beispiel 20

Expression von Creatinase-Protein durch das komplette Promotor-fragment (Promotoren 1 - 7, fnr-Boxen 1 und 2) mit Translationsinitiationsregion des pfl-Gens (translationale Kopplung).

| Stamm: | |
|---|---|
| JM83 (pPFL23A-C) | |
| +O2 | 0,024 |
| -O2 | 0,195 |
| Zellen in der stationären Wachstumsphase geerntet. | |

### Beispiel 21

Expression von Creatinase-Protein durch das verkürzte Promotor-fragment (Promotoren 6 und 7, fnr-Boxen 1 und 2) mit Translationsinitiationsregion des Creatinasegens (transkriptionale Kopplung).

| Stamm: | |
|---|---|
| JM83 (pPFL39-C) | |
| +O2 | 0.006 4) |
| -O2 | 0,045 |

| | |
|---|---|
| 4) Zellen am Anfang der log-Phase geerntet. | |

## Patentansprüche (Patentansprüche für folgende(n) Vertragsstaat(en): AT, BE, CH, LI, DE, DK, FR, GB, GR, IT, LU, NL, SE)

1. Rekombinante DNA,
**dadurch gekennzeichnet**,
daß sie eine Regulatorregion enthält, die zu mindestens 65 % homolog zu den Teilbereichen -969 bis -991 Basenpaare und -1308 bis -1330 Basenpaare der Sequenz von Figur 10 ist und in 3'-Richtung von der Regulationssequenz mindestens eine Promotorregion enthält, welche eine -35/-10-Promotor-Sequenz enthält.

2. Rekombinante DNA nach Anspruch 1,
**dadurch gekennzeichnet**,
daß sie zusätzlich mindestens eine dritte Sequenz enthält, die mindestens zu 80 % homolog zu der folgenden Konsensussequenz ist:

      C  T
TATTTG AT AA
      G  -

3. Rekombinante DNA nach einem der Ansprüche 1 oder 2,
**dadurch gekennzeichnet**,
daß sie als Promotor den lac-, Lambda PL-, trp- oder mgl-Promotor enthalten.

4. Expressionsvektor,
**dadurch gekennzeichnet**,
daß er eine rekombinante DNA gemäß einem der vorhergehenden Ansprüche einligiert in ein geeignetes Vektormolekül enthält.

5. Expressionsvektor nach Anspruch 4,
**dadurch gekennzeichnet**,
daß dieser downstream der rekombinanten DNA einen Polylinker oder eine singuläre Restriktionsschnittstelle zum Einsetzen eines Fremdgens enthält.

6. Verfahren zur Herstellung einer rekombinanten DNA nach einem der Ansprüche 1 bis 3,
**dadurch gekennzeichnet**,
daß man aus der Genbank eines Mikroorganismus, der das pfl-Gen enthält, dieses mit seinen upstream Regionen isoliert und die gewünschten Teile nach an sich bekannten Methoden erhält und mit den gewünschten anderen Sequenzen bzw. Promotoren verbindet.

7. Verfahren zur Herstellung eines Expressionsvektors nach einem der Ansprüche 4 oder 5,
**dadurch gekennzeichnet**,
daß man die isolierte rekombinante DNA-Sequenz gegebenenfalls mit einem Polylinker, einer Shine-Dalgarno-Sequenz, einem Startkodon und/oder eventuellen weiteren Sequenzen in einem geeigneten Vektor insertiert.

8. Verwendung einer rekombinanten DNA nach einem der Ansprüche 1 bis 3 oder eines Expressionsvektors nach einem der Ansprüche 4 oder 5 zur induzierbaren und reprimierbaren Expression eines Fremdgens,
**dadurch gekennzeichnet**,
daß die Induktion unter anaeroben Bedingungen und durch Pyruvat und die Repression durch Sauerstoff bewirkt wird.

## Patentansprüche (Patentansprüche für folgende(n) Vertragsstaat(en): ES)

1. Verfahren zur Herstellung einer rekombinanten DNA, die eine Regulatorregion enthält, die zu mindestens 65 % homolog zu den Teilbereichen -969 bis -991 Basenpaare und -1308 bis -1330 Basenpaare der Sequenz von Figur 10 ist und in 3'-Richtung von der Regulationssequenz mindestens eine Promotorregion enthält, welche eine -35/-1O-Promotor-Sequenz enthält,
**dadurch gekennzeichnet**,
daß man aus der Genbank eines Mikroorganismus, der das pfl-Gen enthält, dieses mit seinen upstream Regionen isoliert und die gewünschten Teile nach an sich bekannten Methoden erhält und mit den gewünschten anderen Sequenzen bzw. Promotoren verbindet.

2. Verfahren nach Anspruch 1,
**dadurch gekennzeichnet**,
daß man zusätzlich eine dritte Sequenz mit den anderen Sequenzen verbindet, welche mindestens zu 80 % homolog zu der folgenden Konsensussequenz ist:

      C  T
TATTTG AT AA
      G  -

3. Verfahren nach Anspruch 1 oder 2,
**dadurch gekennzeichnet**,
daß man als Promotor den lac-, Lambda PL-, trp- oder mgl-Promotor verwendet.

4. Verfahren zur Herstellung eines Expressionsvektors, der eine rekombinante DNA gemäß einem der vorhergehenden Ansprüche einligiert in ein geeignetes Vektormolekül enthält,
**dadurch gekennzeichnet**,
daß man die isolierte rekombinante DNA-Sequenz gegebenenfalls mit einem Polylinker, einer Shine-Dalgarno-Sequenz, einem Startkodon und/oder eventuellen weiteren Sequenzen in einem geeigneten Vektor insertiert.

5. Verfahren nach Anspruch 4,
**dadurch gekennzeichnet**,
daß man zusätzlich downstream der rekombinanten DNA einen Polylinker oder eine singuläre Restriktionsschnittstelle zum Einsetzen eines Fremdgens einfügt.

6. Verwendung einer rekombinanten DNA, hergestellt nach einem der Ansprüche 1 bis 3 oder eines Expressionsvektors, hergestellt nach einem der Ansprüche 4 oder 5 zur induzierbaren und reprimierbaren Expression eines Fremdgens,
**dadurch gekennzeichnet**,
daß die Induktion unter anaeroben Bedingungen und durch Pyruvat und die Repression durch Sauerstoff bewirkt wird.

## Claims (Claims for the following Contracting State(s): AT, BE, CH, LI, DE, DK, FR, GB, GR, IT, LU, NL, SE)

1. Recombinant DNA,
**wherein**
it contains a regulator region which is at least 65 % homologous to the partial regions -969 to -991 base pairs and/or -1308 to -1330 base pairs of the sequence of Figure 10 and in the 3' direction from the regulation sequence contains at least one promoter region which contains a -35/-10 promoter sequence.

2. Recombinant DNA as claimed in claim 1,
**wherein**
it additionally contains at least a third sequence which is at least 80 % homologous to the following consensus sequence:

      G  T
TATTTG AT AA
      C  -

3. Recombinant DNA as claimed in one of the claims 1 or 2,
**wherein**
it contains the lac, lambda PL, trp or mgl promoter as the promoter.

4. Expression vector,
**wherein**
it contains a recombinant DNA according to one of the previous claims which is ligated into a suitable vector molecule.

5. Expression vector as claimed in claim 4,
**wherein**
it contains a polylinker or a single restriction cleavage site for the insertion of a foreign gene downstream of the recombinant DNA.

6. Process for the production of a recombinant DNA as claimed in one of the claims 1 to 3,
**wherein**
the pfl gene is isolated with its upstream regions from the gene bank of a microorganism containing the pfl gene and the desired parts are obtained according to well-known methods and combined with the other desired sequences or promoters.

7. Process for the production of an expression vector as claimed in one of the claims 4 to 5,
**wherein**
the isolated recombinant DNA sequence with, if desired, a polylinker, a Shine-Dalgarno sequence, a start codon and/or further possible sequences is inserted into a suitable vector.

8. Use of a recombinant DNA as claimed in one of the claims 1 to 3 or of an expression vector as claimed in one of the claims 4 to 5 for the inducible and repressible expression of a foreign gene,
**wherein**
the induction is effected under anaerobic conditions and by pyruvate and the repression is effected by oxygen.

## Claims (Claims for the following Contracting State(s): ES)

1. Process for the production of a recombinant DNA which contains a regulator region which is at least 65 % homologous to the partial regions -969 to -991 base pairs and/or -1308 to -1330 base pairs of the sequence of Figure 10 and in the 3' direction from the regulation sequence contains at least one promoter region which contains a -35/-10 promoter sequence,
**wherein**
the pfl gene is isolated with its upstream regions from the gene bank of a microorganism containing the pfl gene and the desired parts are obtained according to well-known methods and combined with the other desired sequences or promoters.

2. Process as claimed in claim 1,
**wherein**
additionally at least a third sequence is combined with the other sequences which is at least 80 % homologous to the following consensus sequence:

G T
TATTTG AT AA
C -

3. Process as claimed in one of the claims 1 or 2,
**wherein**
the lac, lambda PL, trp or mgl promoter are used as the promoter.

4. Process for the production of an expression vector which contains a recombinant DNA according to one of the previous claims which is ligated into a suitable vector molecule
**wherein**
the isolated recombinant DNA sequence is inserted into a suitable vector optionally with a polylinker, a Shine-Dalgarno sequence, a start codon and/or further possible sequences.

5. Process as claimed in claim 4,
**wherein**
it additionally contains a polylinker or a single restriction cleavage site for the insertion of a foreign gene downstream of the recombinant DNA.

6. Use of a recombinant DNA produced as claimed in one of the claims 1 to 3 or of an expression vector produced as claimed in one of the claims 4 or 5 for the inducible and repressible expression of a foreign gene,
**wherein**
the induction is effected under anaerobic conditions and by pyruvate and the repression is effected by oxygen.

## Revendications (Revendications pour l'(les) Etat(s) contractant(s) suivant(s): AT, BE, CH, LI, DE, DK, FR, GB, GR, IT, LU, NL, SE)

1. ADN recombiné caractérisé en ce qu'il contient une région régulateur qui est homologue à raison d'au moins 65% des domaines partiels des paires de bases -969 à -991 et -1308 à -1330 de la séquence de la figure 10 et dans la direction 3' de la séquence de régulation au moins une région promoteur qui contient une séquence promoteur -35/-10.

2. ADN recombiné selon la revendication 1 caractérisé en ce qu'il contient en plus au moins une troisième séquence qui est homologue à raison d'au moins 80% de la séquence consensus suivante:

       C  T
TATTTG AT AA
       G  -

3. ADN recombiné selon l'une des revendications 1 et 2, caractérisé en ce qu'il contient comme promoteur le promoteur lac, PL de lambda, trp ou mgl.

4. Vecteur d'expression caractérisé en ce qu'il contient un ADN recombiné selon l'une des revendications précédentes inséré par ligature dans une molécule vecteur appropriée.

5. Vecteur d'expression selon la revendication 4 caractérisé en ce qu'il contient en aval de l'ADN recombiné un lieur multisite ou un site de restriction singulier pour l'insertion d'un gène étranger.

6. Procédé de préparation d'un ADN recombiné selon l'une des revendications 1 à 3 caractérisé en ce qu'on isole le gène pfl avec ses régions en amont à partir d'une banque de gènes d'un micro-organisme qui le contient, et on obtient les parties voulues selon des méthodes connues en soi et on les lie aux autres séquences voulues ou aux autres promoteurs voulus.

7. Procédé de préparation d'un vecteur d'expression selon l'une des revendications 4 et 5 caractérisé en ce que l'on insère dans un vecteur approprié la séquence d'ADN recombiné isolée, éventuellement avec un lieur multisite, une séquence de Shine-Dalgarno, un codon d'initiation et/ou d'éventuelles autres séquences.

8. Utilisation d'un ADN recombiné selon l'une des revendications 1 à 3 ou d'un vecteur d'expression selon l'une des revendications 4 et 5 pour l'expression inductible et répressible d'un gène étranger caractérisée en ce que l'induction est réalisée dans des conditions anaérobies et par le pyruvate et la répression est réalisée par l'oxygène.

## Revendications (Revendications pour l'(les) Etat(s) contractant(s) suivant(s): ES)

1. Procédé de préparation d'un ADN recombiné qui contient une région régulateur qui est homologue à raison d'au moins 65% des domaines partiels des paires de bases -969 à -991 et -1308 à -1330 de la séquence de la figure 10 et qui contient dans la direction 3' de la séquence de régulation au moins une région promoteur qui contient une séquence promoteur -35/-10 caractérisé en ce qu'on isole le gène pfl avec ses régions en amont à partir de la banque de gènes d'un micro-organisme qui le contient, et on obtient les parties voulues selon des méthodes connues en soi et on les lie aux autres séquences voulues ou aux autres promoteurs voulus.

2. Procédé selon la revendication 1 caractérisé en ce qu'on lie en plus aux autres séquences une troisième séquence qui est homologue à raison d'au moins 80% de la séquence consensus suivante:

       C  T
TATTTG AT AA
       G  -

3. Procédé selon la revendication 1 ou 2, caractérisé en ce que l'on utilise comme promoteur le promoteur lac, PL de lambda, trp ou mgl.

4. Procédé de préparation d'un vecteur d'expression qui contient un ADN recombiné selon l'une des revendications précédentes introduit par ligature dans une molécule vecteur appropriée caractérisé en ce que l'on insère dans un vecteur approprié la séquence d'ADN recombiné isolée éventuellement avec un lieur multisite, une séquence de Shine-Dalgarno, un codon d'initiation et/ou d'éventuelles séquences supplémentaires.

5. Procédé selon la revendication 4 caractérisé en ce que l'on insère en plus en aval de l'ADN recombiné un lieur multisite ou un site de restriction singulier pour l'insertion d'un gène étranger.

6. Utilisation d'un ADN recombiné préparé selon l'une des revendications 1 à 3 ou d'un vecteur d'expression préparé selon l'une des revendications 4 et 5 pour l'expression inductible et répressible d'un gène étranger caractérisée en ce que l'induction est réalisée dans des conditions anaérobies et par le pyruvate et la répression est réalisée par l'oxygène.
